# EUROPEAN PATENT SPECIFICATION

(11) **EP 3 979 971 B1**
(45) Date of publication and mention of the grant of the patent: **25.11.2026**
(21) Application number: 20731344.6
(22) Date of filing: 20.05.2020
(51) Int. Cl.: A61J 1/03, A61J 7/04, B65D 75/32, B65D 83/04, A61J 1/16

(54) **RECONFIGURABLE SMART PACKAGE**
REKONFIGURIERBARE INTELLIGENTE VERPACKUNG
EMBALLAGE INTELLIGENT RECONFIGURABLE

(30) Priority: 07.06.2019 US 201916434981
(43) Date of publication of application: 13.04.2022
(73) Proprietor: DoseCall, LLC, Ft. Lauderdale, FL 33316 (US)
(72) Inventor: MEHREGANY, Mehran, San Diego, California 92127 (US)
(74) Representative: Blumbach · Zinngrebe Patentanwälte PartG mbB
(86) International application number: PCT/US2020/033820
(87) International publication number: WO 2020/247180

(56) References cited:
- WO-A1-2012/111034
- WO-A1-2018/156810
- US-A1- 2016 000 657
- US-A1- 2017 294 105
- US-A1- 2019 107 501
- US-A1- 2019 127 102

## Description

### Cross Reference to Related Applications

### Technical Field

The present disclosure relates to packaging in general, and, more particularly, to smart packaging.

### Background

Health care expectations and expenditures are growing worldwide. There is a strong desire to find ways to improve care outcomes and reduce care cost. Improving medication adherence (i.e., dosing-regimen compliance) is a strategy that holds promise to improve outcomes and reduce care cost. The reason is that, to be effective, prescription and over-the-counter medicines (a.k.a., drugs) require good adherence to a specific dosing regimen-taking the right dosage, with the right frequency (dosing interval), at the right time (dosing window) for the prescribed duration.

Unfortunately, dosing-regimen noncompliance is common and leads to a costly problem in many ways, from driving up health care costs to financial losses to the pharmaceutical industry to serious negative human impacts. When a patient practices poor adherence, efficient recovery is less likely, which lengthens recovery duration and can necessitate additional interventions. As a result, treatment outcome is compromised and care cost is increased.

A major portion of health care cost is due to treatment of chronic diseases. Chronic diseases are illnesses that last more than three months. They cannot be prevented by vaccination, and do not disappear on their own or get cured by medication. As a result, treatment typically deals with the resulting symptoms to ease the discomfort of the patient. Examples of chronic diseases include cardiovascular disease, cancer, diabetes, epilepsy and seizer, obesity, depression, schizophrenia, asthma and chronic obstructive pulmonary disease (COPD) to name a few. Chronic diseases usually lead to comorbidities, e.g., an obese person develops hypertension, diabetes, *etc.*

Chronic diseases are prevalent, particularly among the aging population. Nearly half of Americans have at least one chronic disease, at least half of whom have two or more chronic diseases. As a result, chronic diseases account for ~75% of the total health care expenditure in the United States, which was $3.5 trillion in 2017. Trends are generally similar across the world.

It is common that patients, particularly chronic-disease patients, are on more than one medication-some more than a dozen. Many such medications require multiple doses per day. By way of example, a patient on medications A, B, C, D, E and F has to take: (i) A and C four times a day, *i.e.,* morning, noon, evening and night; (ii) B every other day; (iii) D and E morning and night; and (iv) F at noon. It is clear, therefore, that managing adherence to such dosing regimens is challenging. This challenge increases with number of medications and their respective dosing regimen variations.

One approach to improve adherence has been to package patient medications by dosage, with instructions of what dose to take when. One conventional packaging scheme employs blister cards, wherein each blister on the card contains the medications for each dosing window. These blister cards tend to be larger than blister cards for single medications because the blisters must be large enough to accommodate multiple medications per dose. In addition, such blister cards tend to be configured in a weekly format, e.g., an array of 7x4 blisters.

Another conventional approach to improving medication adherence employs "reminders," which can be automated and delivered in the form of alarms, text message, calls and/or e-mails. Typically, a mobile device (usually a smart phone or tablet) running mobile-application software functions as a user interface for receiving such notifications. Alternatively, medication can be placed in a case/vessel/package that incorporates electronic provisions for tracking adherence and issuing audio/visual notifications. In some cases, the case/vessel/package is wirelessly linked to a mobile device and notifications are provided to the user via the mobile device, on the vessel/package, or both. Approaches such as the above are commonly referred to as "smart packaging".

### Summary

The present disclosure teaches systems and methods that facilitate tracking of adherence to medication-dosing regimens through connected, smart packaging. Smart cases in accordance with the present disclosure are particularly well suited for use with medicines provided in blister-cards, multi-medicine blister cards, and the like.

Like smart cases known in the prior art, smart cases in accordance with the present invention include a sensor system that can monitor the state of the lidding film and/or content in the reservoirs of a blister card; however, prior-art sensor systems are configured to monitor one particular blister card having a specific configuration (e.g., reservoir sizes, shapes, and/or inter-reservoir spacings).

In sharp contrast to the prior art, a smart case in accordance with the present disclosure includes a sensor system that can be used to monitor multiple blister cards that have different configurations. Embodiments in accordance with the present disclosure include a plurality of adapters, each of which can be mounted in the same smart case to enable it to accept any of a set of blister cards having different configurations. In some embodiments, the adapters are annular frames having a uniform outer shape and size and an inner annulus that is based on the lateral extent of a different blister card of the set. In some embodiments, some of the adapters include grids of walls that are arranged to surround each blister on its corresponding blister card of the set to securely locate and hold the blister card in the smart case. Furthermore, in some embodiments, the walls also serve to mitigate optical crosstalk between neighboring reservoirs and sensor elements.

An illustrative embodiment is a smart case that includes a housing that includes an electronics module and a detection module. The electronics module includes electronic circuitry for interfacing to a sensor system for monitoring a blister card and communications circuitry for communicating with an external device, such as a mobile phone, as well as other electronic functionality. The detection module comprises a receiver and the sensor system.

The receiver includes a seat, an adapter, and a frame, where the seat supports the adapter and the blister card, the adapter locates the blister card, and the frame secures the blister card and adapter against the seat. The adapter includes a grid of walls that define cells that substantially match the three-dimensional shape of the reservoirs of the blister card such that the blisters of the blister card removably "nest" in the cells. The walls also function as light barriers that mitigate optical crosstalk between adjacent reservoirs and sensor elements.

In the illustrative embodiment, the sensor system includes a plurality of photodiodes that are arranged in an array on a substrate that resides between the seat and the adapter. The photodiode array is configured such that at least one photodiode is located beneath each reservoir. As a result, when the lidding film of a reservoir is removed and its content is dispensed, its respective photodiode will detect light that passes through the reservoir and generate an output signal that indicates the content has been dispensed.

In some embodiments, a smart case includes a plurality of adapters, each having an inner annulus suitable for a different blister card of a set of blister cards. In some embodiments, each adapter includes a grid of walls and cells, where each arrangement matches the configuration of a different blister card of a set of blister cards. In some embodiments, the sensors system includes a photodiode array having a density that is equal to or greater than the highest reservoir density of the reservoirs of the set of blister cards for which the smart case is operative.

In some embodiments, an adapter comprises walls that include electrodes that define the sensor system, where the electrodes are operative for monitoring the content of each reservoir via electrical capacitance tomography.

In some embodiments, the sensor system includes a focal-plane array that can provide the position of any light incident upon it. As a result, the focal plane array is capable of monitoring the state of a blister card having any configuration.

In some embodiments, the sensor system includes a plurality of connectors that are electrically couplable with an electrically conductive lidding film of a blister card, where the connectors enable monitoring of the state of the lidding film via a tomographic imaging technique, such as electrical resistance tomography (ERT) and electrical impedance tomography (EIT).

The invention is defined by claim 1 and is further developed by the features of the dependent claims.

An embodiment in accordance with the present disclosure is a smart case (500) for monitoring the state of a first blister card (122) comprising a first plurality of blisters (210) and a first lidding film (204) having a first plurality of dispensing regions (212), the first plurality of blisters (210) and first plurality of dispensing regions collectively defining a first plurality of reservoirs (208) that contain first content (208), wherein the first plurality of blisters collectively define a first blister region (218), and wherein the smart case comprises a detection module (106) that includes: a receiver (112) that is configured to locate and secure the first blister card, wherein the receiver is located in a first body portion (P2), and wherein the receiver includes a first adapter (508) having a flange (520) that has an outer perimeter (OP1) and a first inner perimeter (IP1), the outer perimeter being based on the dimensions of the first body portion and the inner perimeter being based on the first blister region; and a sensor system (114) that is configured to monitor the state of at least one of the first lidding film and the first content.

Another embodiment in accordance with the present disclosure is a smart case (500) for monitoring the state of any blister card of a set of blister cards (122), each blister card of the set thereof comprising a plurality of blisters (210) and a lidding film (204) having a plurality of dispensing regions (212), the plurality of blisters (210) and plurality of dispensing regions collectively defining a plurality of reservoirs (208) that contain first content (208), wherein the plurality of blisters collectively define a blister region (218), and each blister card of the set thereof having a different configuration, wherein the smart case comprises: a first body portion (P2) for holding a receiver (112) that is reconfigurable to locate and secure each blister card of the set thereof; the receiver; and a sensor system (114) that is operative for monitoring the state of each blister card of the set thereof.

### Brief Description of the Drawings

**FIG.** 1 depicts a functional block diagram of a medicine case in accordance with the present disclosure.
**FIG. 2A** depicts a schematic drawing of a perspective view of blister card **122.**
**FIG. 2B** depicts a schematic drawing of a sectional view of an exemplary reservoir of blister card **122.**
**FIGS. 2C-D** depict plan view schematic drawings of the front (forming-film side) and back (lidding-film side) of blister card **122.**
**3A-B** depict schematic drawings of perspective-top and plan-bottom views, respectively, of case **100.**
**FIG. 3C** depicts a schematic drawing of an exploded sectional view of receiver **112,** sensor system **114,** and blister card **122.**
**FIG. 3D** depicts a schematic drawing of top view of sensor system **114.**
**FIGS. 3E-F** depict schematic drawings of examples of electronically actuatable latches suitable for use in accordance with the present disclosure.
**FIG. 4A** depicts a schematic drawing of a perspective top view of an alternative embodiment of a smart case in accordance with the present disclosure.
**FIG. 4B** depicts a schematic drawing of a sectional view of a portion of detection module **402.**
**FIG. 5A** depicts a schematic drawing of a sectional view of another alternative detection module in accordance with the present disclosure.
**FIG. 5B-C** depict top and perspective views, respectively, of adapter **508.**
**FIGS. 5D-E** depict schematic drawings of an alternative adapter in accordance with the present disclosure.
**FIG. 6A** depicts a schematic drawing of a sectional view of yet another alternative detection module in accordance with the present disclosure.
**FIG. 6B** depicts detection module **600** with the addition of optional cover **606.**
**FIG. 7A** depicts a schematic drawing of an exploded perspective view of the salient features of yet another alternative smart case in accordance with the present disclosure.
**FIGS. 7B-C** depict schematic drawings of exploded perspective views of smart case **700** configured for use with blister cards that are larger and smaller, respectively, than blister card **122.**
**FIGS. 8A-B** depict exploded schematic perspective drawings of two alternative receivers in accordance with the present disclosure.

### Detailed Description

It should be noted that, although embodiments in accordance with the present disclosure are described herein with respect to medications as an exemplary application, concepts of the present disclosure are applicable to a wide range of other applications; therefore, the use of medication should not be viewed as limiting the scope of the utility of the concepts described herein. Other examples of applications in which the smart packaging concepts of the present disclosure can be used include, without limitation, other forms of medication, non-medication products (e.g., vitamins, supplements, *etc.),* foodstuff, gum, beverages, toiletry and beauty care products, razor blades, consumer electronics, toner cartridges, toys, tools, *etc.* In other words, the teachings of the present disclosure are applicable to a wide range of product-tracking applications.

Accordingly, for the purposes of this Specification, including the appended claims, the term **"content"** is used as a general term that encompasses all products packaged in blister-card format.

Furthermore, although the examples described herein are directed to monitoring the state of blister cards containing medicine in tablet form, many types of medication and non-medication (e.g., vitamins, supplements, breath freshener, *etc.)* are offered in forms other than that of a tablet but that are suitable for packaging in a blister card, such as capsules, lozenges, powders, gels, liquids, *etc.* For the purposes of this Specification, including the appended claims, the term **"tablet"** is used as a general term that encompasses all such medicinal and non-medicinal forms.

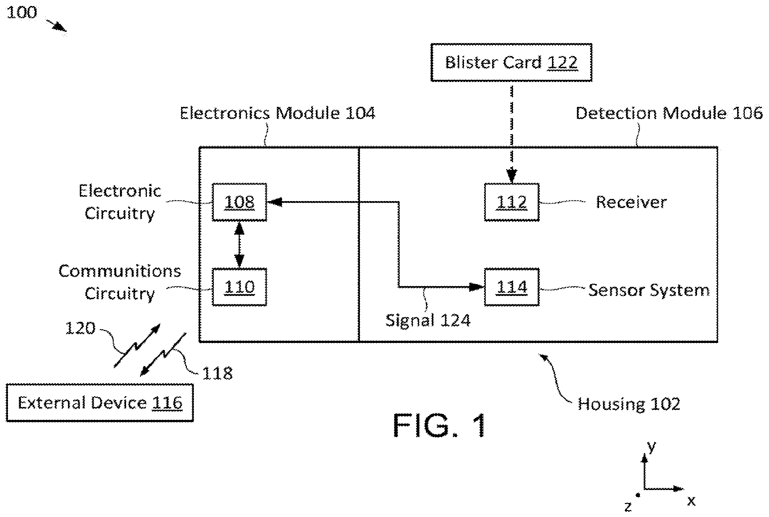

**FIG. 1** depicts a functional block diagram of a medicine case in accordance with the present disclosure. Case **100** includes housing **102,** electronics module **104,** and detection module **106.**

Housing **102** is a non-modular, molded plastic case having a first portion that contains electronics module **104** and a second portion that contains detection module **106.** The second portion includes a body and a lid that can be opened and closed to enable insertion of blister card **122** and then control access to it. In the depicted example, the body and lid are connected along one edge via a flexible hinge portion. As discussed below, the body also includes an electronically actuatable clasp that engages a catch on the lid for controlling access to the blister card.

It should be noted that, although case **100** is a non-modular case that includes non-separable electronics and detection modules, the teachings of the present disclosure are applicable to smart cases having electronics and detection modules that are separable and the description of a non-modular case should not be considered limiting to the scope of the appended claims. Examples of modular smart cases in accordance with the present disclosure are described in U.S. Patent Application Ser. No. 16/333,021.

Electronics module **104** is a module that contains electronics circuitry **108** for interfacing to sensor system **114,** as well as communications circuitry **110.** Electronics module **104** is analogous to electronics modules described in detail in the U.S. Patent Application Ser. No. 14/879,874, U.S. Patent Application Ser. No. 15/170,121, U.S. Patent Application Ser. No. 15/223,779, and U.S. Patent Application Ser. No. 16/333,021.

Electronic circuitry **108** enables the electronics module to receive and condition *(e.g.,* provide pre-amplification, digitization, *etc.)* one or more signals from sensor system **114** of detection module **106,** provide power conditioning and management, display information to the user, *etc.* In some embodiments, electronic circuitry **108** includes one or more of the following:
i. controllers/processors; or
ii. memory; or
iii. sensor circuitry; or
iv. power circuitry; or
v. display circuitry and/or one or more displays; or
vi. sleep-mode circuitry; or
vii. energy-storage systems; or
viii. clock circuitry; or
ix. energy-scavenging systems; or
x. sensor-interface circuitry; or
xi. on-case alerts (e.g., LCD displays, speakers, buzzers, *etc.);* or
xii. sensors, such as environmental (e.g., touch, temperature, acceleration, humidity, shock, geolocation, *etc.),* biosensors (e.g., bioimpedance, biopotential, breath analyzer, alcohol detector, *etc.),* and the like; or
xiii. any combination of i, ii, iii, iv, v, vi, vii, viii, ix, x, xi, and xii.

Communications circuitry **110** enables communication with external device **116.** In the depicted example, communications circuitry **110** includes a wireless Bluetooth Low-Energy (BLE) transceiver for transmitting operational communications **120** and receiving status signal **118** to/from external device **116.** In some embodiments, communications circuitry includes a different wired and/or wireless communications electronics, such as FireWire, USB, lightning connector, a dock connector, cellular, WiFi, near-field-communications (NFC) radio, optical links, *etc.*

Detection module **106** comprises receiver **112** and sensor system **114.** Receiver **112** includes fixturing that is configured to position and hold blister card **122** in a location that enables its state to be monitored by sensor system **114.** Detection module **106** is described in detail below.

In the depicted example, external device **116** is a smart phone that communicates with electronics module **104** via communications circuitry **110** and runs a software application (i.e., a mobile app) that provides assistance to the patient and/or caregiver to achieve and maintain good adherence to the prescribed drug regimen. In some embodiments, external device **116** is a different device, such as a different mobile device (e.g., smart watch, computer tablet, *etc.),* a computer, a gateway and/or a base station. Preferably, the software application running on external device **116** is supported by data analytics, which include artificial intelligence and machine learning. In some embodiments, external device **116** is connected to the cloud backend through wireless and/or wired communications.

The smart case and smartphone app determine the state of the blister card and provide a visual and/or audible indication of adherence. If failure to follow the regimen is detected, the smartphone contacts one or more people in the user's defined support group (e.g., a caregiver, spouse, child, doctor, *etc.)* to alert them that the user might require assistance.

In the depicted example, case **100** is a smart case for tracking the state of blister card **122,** which contains medicinal content for a one-week medicinal regimen having four dosing periods (A through D) for each of seven days (1 through 7). In physical form, smart case **100** is analogous to those described in detail in the '874, '121, '779, and '021 applications. It should be noted that, while the depicted example is configured to monitor the state of a blister card having a 7x4 dosing arrangement, the principles of the present disclosure can be applied to a smart case suitable for use with any practical blister card arrangement and dosing schedule. For example, a 7x4 blister card may correspond to a single daily dose over 4 weeks, and a 4x1 blister card may correspond to a single daily dose over 4 days or a weekly dose over 4 weeks. Furthermore, the geometric placement of blister reservoirs may vary depending on human factors related to different use cases.

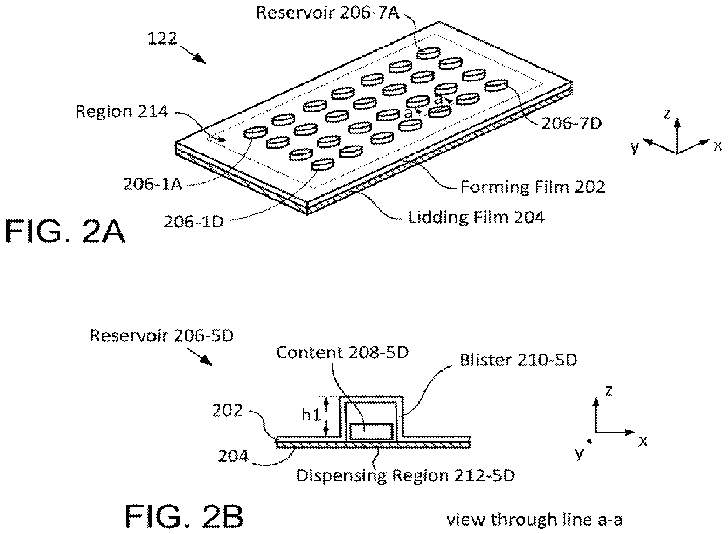

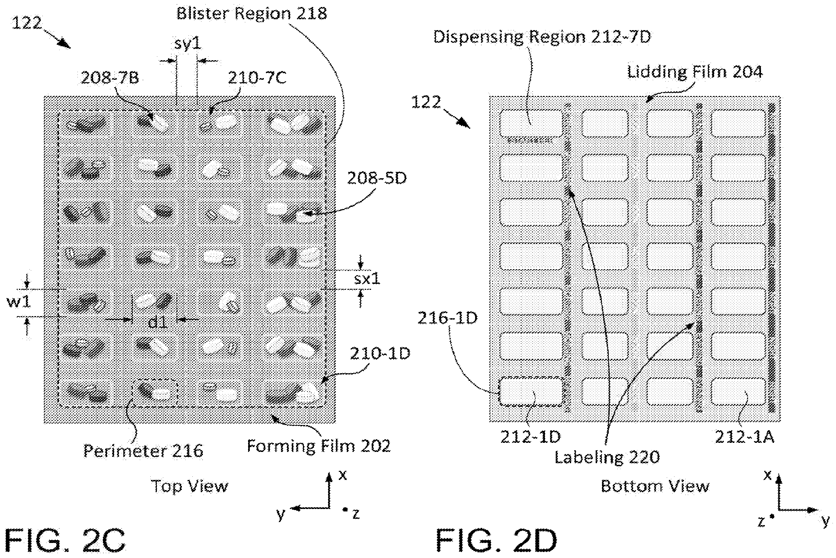

**FIG. 2A** depicts a schematic drawing of a perspective view of blister card **122.**

**FIG. 2B** depicts a schematic drawing of a sectional view of an exemplary reservoir of blister card **122.** The view depicted in FIG. 2B shows a cross-sectional view of reservoir **206-5D,** which is taken through line a-a as shown in FIG. 2A. Reservoir **206-5D** is representative of each of reservoirs **206-1A** through **206-7D.**

**FIGS. 2C-D** depict plan view schematic drawings of the front (forming-film side) and back (lidding-film side) of blister card **122.**

Blister card **122** includes forming film **202** and lidding film **204,** which are configured to define reservoirs **206-1A** through **206-7D.** Reservoirs **206-1A** through **206-7D** contain content **208-1A** through **208-7D,** respectively, each of which is the proper dose for a specific dosing window of a one-week medicinal regimen. In the depicted example, reservoirs **206-1A** through **206-7D** (referred to, collectively, as "reservoirs **206")** are arranged in a seven-row, four-column format.

Forming film **202** is a layer of thermoformed plastic in which cavities for holding medicinal content are formed, thereby defining blisters **210-1A** through **210-7D** (referred to, collectively, as blisters **210).**

In the depicted example, lidding film **204** is a thin sheet of aluminum foil. In some embodiments, lidding film **204** is a sheet of different electrically conductive material. In some embodiments, lidding film **204** includes a sheet of electrically conductive material and a sheet of electrically insulating material, such as a paper sheet (with a printed calendar or instructions), polymer, *etc.* on the bottom side of the lidding film and/or sandwiched between the lidding and forming films. In some embodiments, lidding film **204** is a sheet of electrically non-conductive material (e.g., paper, *etc.)* or a laminate of electrically non-conductive materials (e.g., paper and polymer, *etc.).*

After content **208-1A** through **208-7D** (referred to, collectively, as content **208)** is dispensed into blisters **210,** lidding film **204** is joined with forming film **202** to seal the cavities, thereby completing reservoirs **206** and enclosing content **208.** The portions of lidding film **204** under blisters **210-1A** through **210-7D** define dispensing regions **212-1A** through **212-7D,** respectively, through which content **208-1A** through **208-7D** is dispensed. Dispensing regions **212-1A** through **212-7D** (referred to, collectively, as dispensing regions **212)** are all located within two-dimensional region **214.** Each dispensing region **212** is characterized by perimeter **216,** which is defined by the annulus at which its respective blister **210** is joined with lidding film **204.**

Each of blisters **210** is characterized by a width, **w1,** in the x-direction, a depth, **d1,** in the y-direction, and a height, **h1,** in the z-direction. Blisters **210** are spaced apart by inter-reservoir spacings **sx1** and **sy1** in the x- and y-directions, respectively. For multi-medicine blister cards, at least one of the width, depth, height, and spacings of blisters **210** can be non-uniform over the blister card.

Perimeters **216** collectively define blister region **218,** which is a two-dimensional region whose lateral extent is defined by the outer edges of the perimeters **216** of the outer rows and columns of blisters **212.**

Blister card **122** also includes labeling **220** for providing dosing regimen information directly on the back of the blister card. In some cases, labeling **220** includes other information, such as a calendar that denotes the drug regimen, manufacturing information (date, lot number, *etc.),* and the like.

In some embodiments, case **100** is configured to monitor the state of a blister card in which at least one reservoir contains content other than a plurality of tablets. In some embodiments, some reservoirs may be left empty, for example, to simply maintain a habitual regimen routine or accommodate dosing schedules that do not use all of the reservoirs of a given blister card format. In some embodiments, one or more of such empty reservoirs may not have an intact lidding film or a lidding at all.

As will be apparent to one skilled in the art, at least some of reservoirs **206** are larger than those of single-medication blister cards to enable them to hold medicinal content for doses that include multiple tablets. An exemplary multi-tablet reservoir has a volume of 2 cubic inches, with a dispensing region **212** of approximately 1x2 inches and a blister height of approximately 1 inch. The spacing between reservoirs **206** (i.e., the inter-reservoir spacing of blister card **122)** in each of the x- and y-dimensions is 1/4 inch, wherein 1 inch corresponds to 2.54 cm. As a result, a 7x4 format blister card size populated with such reservoirs requires that region **214** be about 10x10 inches.

It is an aspect of the present disclosure that the teachings herein enabling monitoring of any blister card.

One skilled in the art will recognize, after reading this Specification, that the design features of smart case **100** described herein are based on the particular arrangement of blister card **122,** as well as the sensing technology used to monitor its state and, as a result, are merely exemplary.

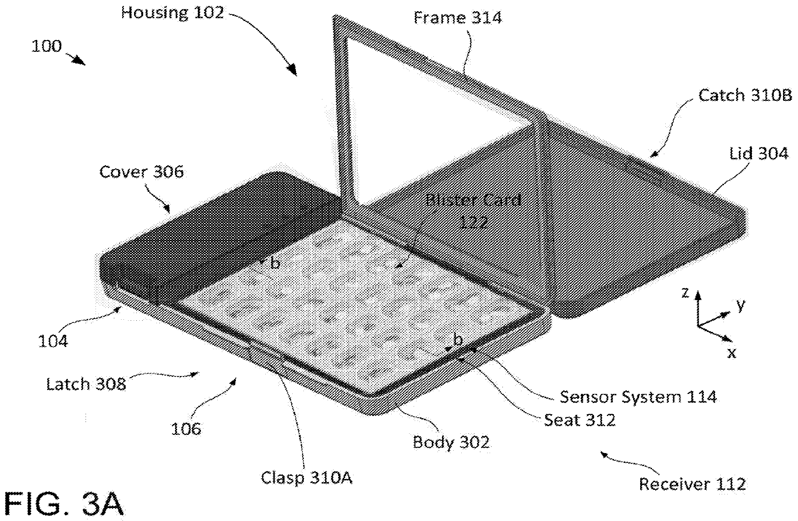

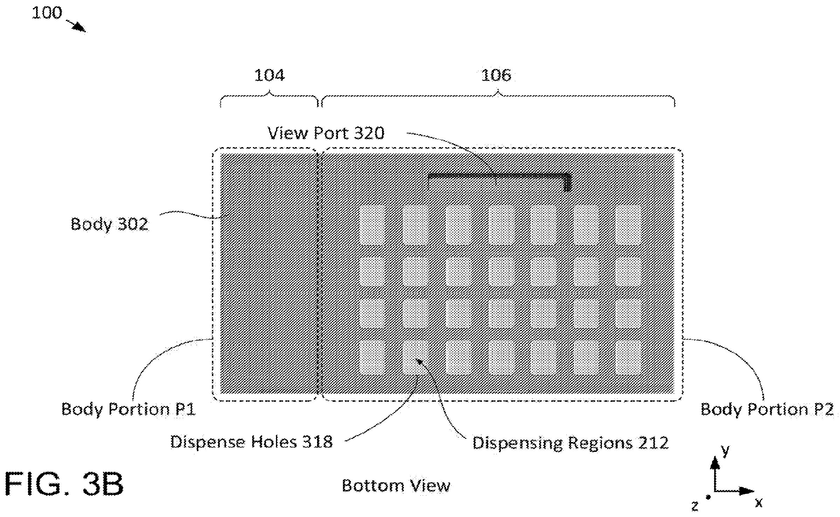

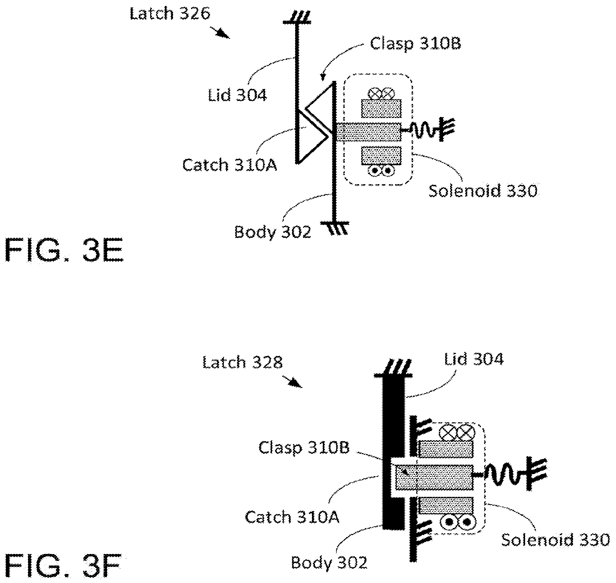

**FIGS. 3A-B** depict schematic drawings of perspective-top and plan-bottom views, respectively, of case **100.**

Housing **102** includes body **302,** lid **304,** and cover **306.** Body **302** extends along the entire bottom side of case **100.** Cover **306** is rigidly affixed to body **302** to enclose and protect the components of electronics module **102,** while lid **304** is a movable "clamshell" cover that is attached to body **304** via a hinge (not shown) along the length of detection module **106.** As a result, lid **304** can be opened to enable access to content **208** and closed to protect blister card **122.**

Body **302** includes body portions **P1** and **P2,** which hold electronics module **104** and detection module **106,** respectively. Body portion **P2** includes dispense holes **318** and view port **320.**

In the depicted example, since case **100** is non-modular, body portions **P1** and **P2** are contiguous sections of unitary body **302.** As will be apparent to one skilled in the art, after reading this disclosure, for embodiments wherein a smart case is modular, body portions **P1** and **P2** are typically separate elements that can be reversibly connected as described in '021 application.

Dispense holes **318** are configured to enable passage of their respective content **208** through body portion **P2** when dispensed.

View port **320** is an opening in body portion **P2** that is configured to expose a portion of blister card **122** for viewing. View port **320** is optionally included to enable information printed on a blister card (such as a bar code, *etc.)* to be read through body **302.**

In the depicted example, housing **102** includes latch **308,** which is configured to reversibly secure lid **304** and body **302** in a closed configuration. Latch **308** comprises clasp **310A** disposed on body **302** and catch **310B** disposed on lid **304.** To unlock latch **308** and enable lid **304** to move relative to body **302,** the user mechanical decouples clasp **310A** and catch **310B.** A wide range of conventional mechanical latching designs can be used for latch **308,** including passive designs based on magnets, zippers, Velcro, sticky films, *etc.*

In the depicted example, receiver **112** includes seat **312** and frame **314.**

Seat **312** is the bottom portion of body **302** and is configured to accept sensor system **114** and blister card **122.** Seat **312** includes dispense holes **318,** which have a one-to-one correspondence with reservoirs **206.**

Frame **314** is a rigid annular element that includes a large central opening configured to expose all of reservoirs **206** when blister card **122** is installed in case **100.** Frame **314** is rotatable about a hinge (not shown) such that it can be closed over blister card **122** and latched in place. Once frame **314** is closed and latched, the blister card is securely held in an operatively coupled arrangement with sensor system **114,** which is disposed on seat **312.** In some embodiments, frame **314** includes a plurality of openings, each of which is configured to expose one or more reservoirs. In some embodiments, frame **314** is separately placed over the blister card after the blister card is inserted. In such embodiments, frame **314** is typically reversibly affixed in place by mechanical, magnets, electromagnets, latches and the like.

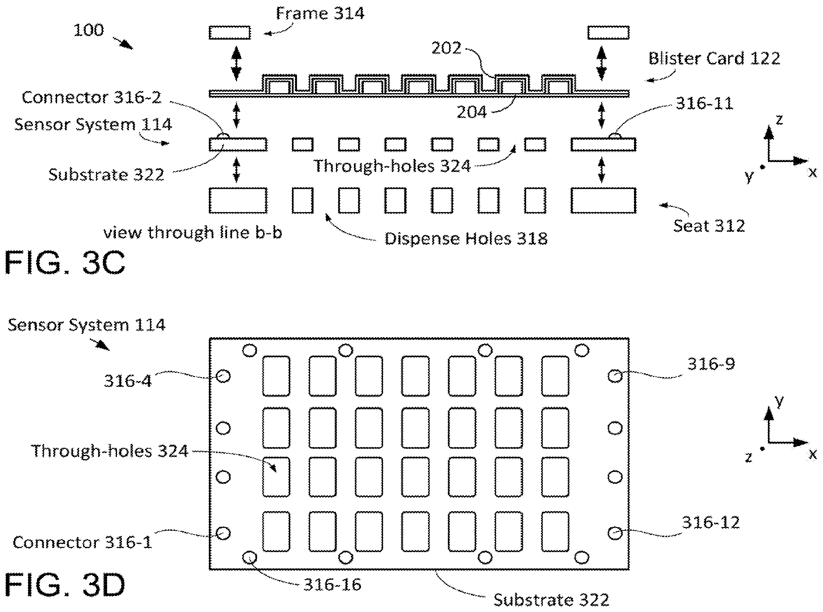

**FIG. 3C** depicts a schematic drawing of an exploded sectional view of receiver **112,** sensor system **114,** and blister card **122.** The view depicted in FIG. 3C is taken through line b-b shown in FIG. 3A.

**FIG. 3D** depicts a schematic drawing of top view of sensor system **114.** Sensor system **114** includes substrate **322,** through-holes **324,** and connectors **316-1** through **316-16.**

Sensor system **114** includes connectors **316-1** through **316-16,** which are configured to electrically couple with lidding film **204** and provide signal **124** to electronic circuitry **108.** Sensor system **114** enables monitoring of the state of lidding film **204** via electrical resistance tomography (ERT) imaging, which is typically performed by electronic circuitry **108.** As will be appreciated by one skilled in the art and as discussed in the '603 application, ERT imaging of lidding film **204** can be used to detect that a hole has been formed in a dispensing region **212** and where the hole is located. As a result, sensor system **114** can determine when one of content **208-1A** through **208-7D** has been dispensed and identify which dose was dispensed. In some embodiments, a different tomographic imaging technique is used (such as electrical impedance tomography (EIT), electrical capacitance tomography (ECT), and the like) to accommodate lidding films that are not electrically conductive.

Each of connectors **316-1** through **316-16** (referred to, collectively, as connectors **316)** is a contact bump disposed on the top surface of substrate **322** and configured to enable blister card **122** to be operatively coupled with sensor system **114** without significant deformation of the blister card. Connectors **316** are electrically coupled with electronics module **104** via conventional electrical traces (not shown) disposed on substrate **322.**

In some embodiments, connectors **316** are other than contact bumps, such as leaf-spring "wiper" contacts, "zero-insertion-force" (ZIF) connectors that close over the blister card after it has been inserted, and the like. In some embodiments, connectors **316** are configured to contact an electrically conductive lidding film capacitively (e.g., from the lidding film side, through an insulating layer disposed on the bottom side of lidding film **204,** through the forming film when connectors **316** are placed on the forming-film side of the blister card **122,** *etc.).* One skilled in the art will recognize, after reading this Specification, that the geometrical shapes and distributions of such resistive or capacitive contacts can be varied substantially to meet a wide range of performance specifications.

Substrate **322** is a printed-circuit board (PCB) that incorporates through-holes **324,** which are configured to enable the passage of content **208** through the substrate **322** when the content is dispensed. It should be noted that, although the depicted example comprises a substrate **322** that is a PCB, myriad alternative materials can be used for substrate **322** without departing from the scope of the present invention.

Although in the illustrative embodiment sensor system **114** is an optical-sensor-based system, in some embodiments, sensor system **114** is based on a different sensing technology , wherein a plurality of sensors (e.g., capacitive sensors, strain sensors, optical sensors, acoustic sensors, tactile sensors, resistance sensors, impedance sensors, thermal sensors, magnetic sensors, *etc.)* having a one-to-one correspondence with dispensing regions **212-1A** through **212-7D** are employed. Examples of sensors suitable for use in such sensing schemes are described in detail in the '874, '121, '779, and '021 applications.

It is well understood that some medications, such as opioids, are highly addicting. It is an aspect of the present disclosure that access to such medication can be controlled via a smart case that includes an electrically-actuated latch that enables a dosing regimen to be programmed such that the smart case can only be opened during a dosing window. Such embodiments offer the promise of mitigating medication abuse by the user. Furthermore, if a user removes more doses than called for during an approved dosing window, it can be detected by case **100** (including if the blister card is completely removed) and an alert can be immediately issued to an appropriate party.

**FIGS. 3E-F** depict schematic drawings of examples of electronically actuatable latches suitable for use in accordance with the present disclosure.

Each of latches **326** and **328** comprises catch **310A,** clasp **310B,** and solenoid **330.**

Solenoid **330** is a conventional solenoid that is mechanically coupled with clast **310B.** Solenoid **330** is configured to disengage clasp **310B** from catch **310A** only in response to a command from electronics module **104,** thereby unlocking body **302** from lid **304** to enable access to content **208.** In some embodiments, latch **308** can only be actuated for an authorized user (as determined via a user-entered access code, fingerprint, audio or image identification, *etc.)* and/or only during an appropriate dosing window. In such embodiments, case **100** provides a measure of protection against an accidental ingestion of content **208** *(e.g.,* by a child, *etc.),* overmedication or abuse by the user, and the like.

In some applications, pushing content contained in a reservoir through its respective dispensing region and dispense hole is potentially problematic. For example, in the depicted example, one or more tablets might break when forced against one another as content **208** is dispensed from multi-tablet reservoir **206.** The potential for tablet breakage increases with the number of tablets within the reservoir - particularly as the height and/or volume of the reservoir increases to accommodate more tablets and/or tablets are themselves fragile. Tablet breakage is also an issue for large disk-shaped tablets - even when contained in individual, conformal reservoirs.

As a result, in some cases, it is preferred that the content of a reservoir can be accessed by removal of at least a portion of its respective dispensing region instead of pushing the content through it. In such cases, blister card **122** is typically held in case **100** such that its lidding film faces toward the user. Once a sufficient portion of a dispensing region **212** is removed, the user can remove its respective content manually or turn the blister card over to enable the tablets to drop out of reservoir **206.**

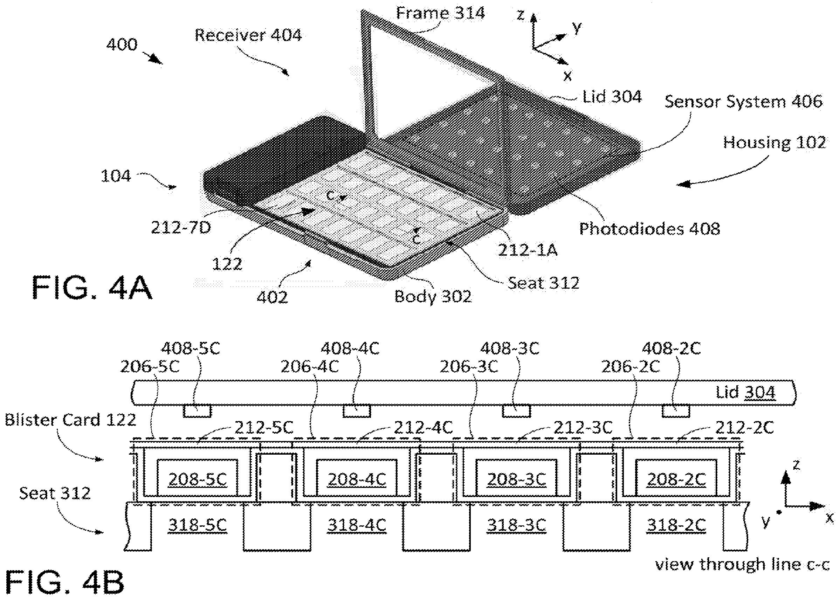

**FIG. 4A** depicts a schematic drawing of a perspective top view of an alternative embodiment of a smart case in accordance with the present disclosure.

Case **400** includes housing **102,** electronics module **104,** and detection module **402.** Case **400** is analogous to case **100;** however, case **400** is configured to monitor a blister card that is held with its lidding film facing lid **304,** includes a sensor system that has a one-to-one correspondence with reservoirs **206,** and such that the sensor system is disposed on the interior surface of lid **304.**

**FIG. 4B** depicts a schematic drawing of a sectional view of a portion of detection module **402.** The sectional view shown in FIG. 4B is taken through line c-c provided in FIG. 4A. Detection module **402** includes receiver **404** and sensor system **406.**

Receiver **404** includes seat **312** and frame **314.** Receiver **404** is analogous to receiver **112;** however, receiver **404** is configured to position blister card **122** in an upside-down orientation (i.e., lidding film **204** is distal to seat **312)** and such forming film **202** is disposed directly on seat **312.** As a result, reservoirs **206** are aligned with dispense holes **318,** which serve as viewing holes that enable the user to see content **208.** In some embodiments, dispense holes **318** facilitate registration of the lateral position of blister card **122** by capturing a portion of its blisters.

For operation with a blister card whose lidding film includes printed instructions, it is preferable that frame **314** is optically transparent, or incorporates openings, to expose the information.

Sensing system **406** comprises photodiodes **408-1A** through **408-7D** (referred to, collectively, as photodiodes **408).** Photodiodes **408** have a one-to-one correspondence to reservoirs **206-1A** through **206-7D** and are disposed on the interior surface of lid **304.** Sensor system **406** also includes conventional electrical connections from photodiodes **408** to enable them to provide signal **124** to electronics module **104** (not shown), such as flexible ribbon cables that pass through the hinge cavity.

In operation, case **400** is opened to provide access to dispensing regions **212,** enabling the user to remove the proper content from blister card **122** by removing at least some of the dispensing region from its reservoir.

Once the proper dose has been dispensed, lid **304** is closed. Since the lidding film material in the dispensing regions of dispensed content is missing, light can pass through these reservoirs to be detected by those photodiodes **408** aligned with them. These photodiodes provide output signals to electronics module **104,** which determines whether a change in the output-signal configuration has occurred, thus indicating which content was dispensed. Since reservoirs whose dispensing regions are still intact will substantially block light, their respective photodiodes generate substantially no output signal.

When the light incident on a photodiode **408** is at a maximum, its respective reservoir is determined to be empty. When no light is incident on a photodiode **408,** its respective reservoir is determined to be untouched (i.e., full). In some cases, however, a partial dose is left in a reservoir after its dispensing region has been opened. This affects the amount of light incident on its respective photodiode **408,** thereby providing an indication of an incomplete dosing event. In addition, a sensor system that can detect the magnitude of light received by a photodiode enables monitoring of a blister card whose lidding film is optically transparent (e.g., a thin film of nylon, clear plastic, *etc.).* In some embodiments, machine learning and artificial intelligence enables a light signal whose intensity is somewhere between zero and the maximum to be related to the amount of content remaining in a reservoir (e.g., tablet types, remaining quantity, *etc.).*

In the depicted example, the light that passes through dispense holes **318** is ambient light. In some embodiments, one or more light sources are included in case **400** (e.g., disposed on seat **312)** to provide the light detected by photodiodes **408.** In some embodiments, each reservoir is provided a different light source. In such embodiments, the inclusion of dispense holes **318** in body **302** is optional.

It should be noted that, while sensing system **406** includes sensors that employ optical-sensing technology, cases in accordance with the present disclosure can employ sensing systems that are based on any of a wide range of sensing technologies. Sensing technologies that can be employed in embodiments in accordance with the present disclosure include, without limitation, capacitive sensing, strain sensing, optical sensing, acoustic sensing, tactile sensing, resistance sensing, impedance sensing, thermal sensing, magnetic sensing, and tomographic imaging. Examples of alternative sensing approaches suitable for use in accordance with the present disclosure are described in detail in the '874, '121, '779, and '021 applications.

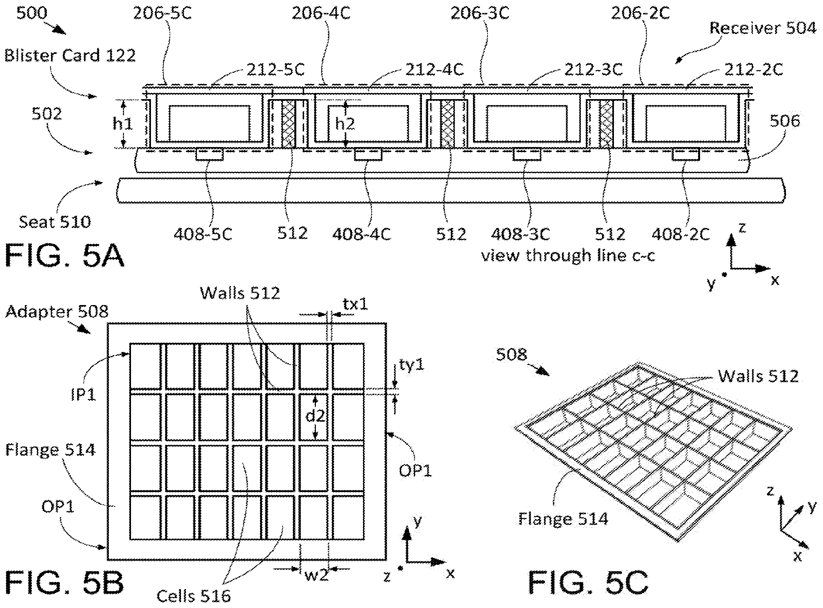

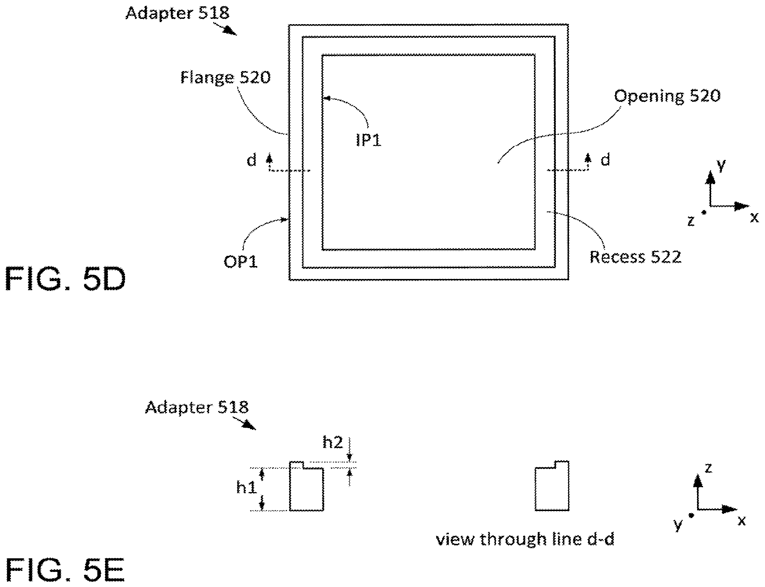

**FIG. 5A** depicts a schematic drawing of a sectional view of another alternative detection module in accordance with the present disclosure. The sectional view shown in FIG. 5A is analogous to the sectional view shown in FIG. 4B. Detection module **500** includes sensor system **502** and receiver **504.** Case **500** is analogous to case **400;** however, in case **500,** sensor system **502** is a photodiode-based sensor system that is disposed underneath blister card **122.** In addition, receiver **504** includes optional adapter **508,** which includes a grid of walls that function as light-blocking features between adjacent reservoirs **206.**

Sensor system **502** is analogous to sensor system **406;** however, in sensor system **502,** photodiodes **408** are located on substrate **506.**

Substrate **506** is a PCB on which photodiodes **408** are mounted. In some embodiments, substrate **506** is another suitable substrate on which photodiodes **408** are disposed. In some embodiments, photodiodes **408** are monolithically integrated on or in substrate **506.** In some embodiments, substrate **506** is an organic or an inorganic semiconductor facilitating the fabrication of photodiodes **408.**

Sensor system **502** is configured to interrogate the lidding film of blister card **122** while lid **304** is open by virtue of photodiodes **408,** which detect ambient light that passes through reservoirs **206** whose dispensing regions **212** have been removed. In some embodiments, however, one or more light sources are disposed on the interior surface of lid **304** to provide light that can be detected by photodiodes **408** when the lid is closed.

Receiver **504** includes seat **510,** frame **314** (not shown), and optional adapter **508.**

Seat **510** mechanically supports sensor system **502,** adapter **508,** and blister card **122.** In the depicted example, seat **510** is the bottom of body **302.** It should be noted that, since content **208** is dispensed from the top side of detection module **500,** seat **510** does not require dispense holes **318.**

In some instances, light can leak from an opened reservoir to the photodiode of a neighboring reservoir (referred to as "optical crosstalk"), which can cause problems. For example, optical crosstalk can lead to confusion regarding which of content **208-1A** through **208-7D** has been dispensed, or corrupt the analysis used to determine the amount and type of content remaining in a partially empty reservoir.

In the depicted example, therefore, the inclusion of optional adapter **508** in receiver **504** affords significant advantages over prior-art smart cases. First, the walls of the adapter substantially block light from passing between adjacent reservoirs of a blister card, thereby mitigating optical crosstalk between neighboring reservoirs and photodiodes. Second, as discussed below, adapters can enable the same smart case to accommodate a wide range of blister cards having different configurations. In some unclaimed embodiments, adapter **508** is not included.

**FIG. 5B-C** depict top and perspective views, respectively, of adapter **508.** Adapter **508** comprises walls **512** and flange **514.** In the depicted example, adapter **508** is made of injection-molded plastic and is substantially optically opaque. In some embodiments, adapter **508** is made of a different suitable opaque material (e.g., metal, ceramic, *etc.).*

Adapter **508** comprises a grid of orthogonal walls **512** having height, **h1,** which is equal to the height, **h1,** of blisters **210** so that adapter **508** provides mechanical support for blister card **122.** Walls **512** collectively define cells **516,** which are characterized by a width, **w2,** in the x-direction, a depth, **d2,** in the y-direction, and a height, **h2,** in the z-direction. Typically, at least one of the dimensions of cell **516** is based on a dimension of blister **210.** In the depicted example, cells **516** have substantially the same three-dimensional shape as blisters **210;** therefore, all three dimensions of the cells match those of the blisters (i.e., **w2=w1, d2=d1,** and **h2=h1).** As a result, cells **516** capture blisters **510,** thereby restricting the lateral movement of blister card **122.**

Flange **514** is an annular plate that extends outward from walls **512** at the top of adapter **508.** Flange **514** has outer perimeter **OP1** and inner perimeter **IP1.** The value of **OP1** is selected based on the dimensions of body portion **P2** and, typically, is selected to enable adapter **508** to fit snugly within body portion **P2.** The value of **IP1** is selected to enable all of blisters **210** of blister card **122** to fit within the flange. In the depicted example, **IP1** is configured to match the perimeter of blister region **218.** In some embodiments, **IP1** is configured to be larger than the perimeter of blister region **218,** as discussed above and with respect to FIG. 2C.

In the depicted example, adapter **508** is integral (i.e., non-removable) to detection module **500;** however, in claimed embodiments the adapter is removable such that different adapters can be used with the same smart case, thereby enabling the case to be used with a set of blister cards having different configurations. Adapter **508** can be removably secured in the detection module using a variety of latching schemes (snapping, sliding, locking, magnetic, electromagnetic, *etc.).*

It should be noted that walls **512** of adapter **508** are substantially straight and vertical and have thicknesses, **tx** and **ty,** in the x- and y-directions, respectively, that are based on the inter-reservoir spacings **sx1** and **sy1** between blisters **210.** In some embodiments, walls **512** have a shape that affords additional functionality, however.

**FIGS. 5D-E** depict schematic drawings of an alternative adapter in accordance with the present disclosure. Adapter **518** is an annular element having flange **520** and opening **522.**

Flange **520** is analogous to flange **514** and has an outer perimeter **OP1** that enables it to fit snugly within body portion **P2.**

Opening **520** has an inner perimeter **IP1** that substantially matches the perimeter of blister region **218.** As a result, blisters **210** of blister card **122** fits snugly into opening **520,** thereby mitigating lateral movement of the blister card relative to flange **520.** In some embodiments, opening **520** is larger in at least one dimension than blister region **218.**

Flange **520** also includes optional recess **522,** which has a perimeter that substantially matches the perimeter of blister card **122** and a height, **h2,** that is equal to the combined thickness of forming film **202** and lidding film **204.** As a result, the blister card sits snugly within recess **522** even when opening **520** is larger than blister region **218.**

In the depicted example, the height of recess **522** above the bottom of flange **520** is equal to the height, **h1,** of blisters **210.** In some embodiments, however, recess **522** resides at a different height above the bottom of flange **520.**

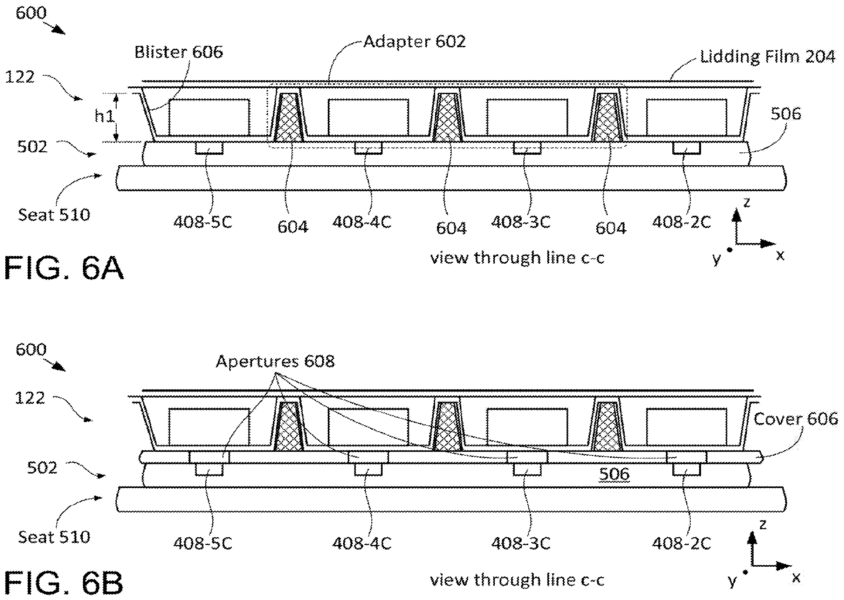

**FIG. 6A** depicts a schematic drawing of a sectional view of yet another alternative detection module in accordance with the present disclosure. Detection module **600** is analogous to detection module **500;** however, detection module **600** includes an adapter whose walls conform to the shape of the blisters of the blister card for which it is intended. Adapter **602** includes flange **514** and walls **604.**

Walls **604** of adapter **602** are tapered to conform to the shape of blisters **606,** which have sidewalls that taper inward from lidding film **204** (i.e., the top of the blister is narrower than its base where it meets the lidding film). In others words, walls **602** define cells that intimately surround blisters **606.** As a result, the large contact area between the blisters **606** and walls **604** enable conformal walls **604** to better "grip" the blister card and keep it in its desired location. The grip between the walls and blisters can be further enhanced by walls having a tacky surface.

It should be noted that walls **512** and **604** represent just two exemplary shapes of adapter walls that are within the scope of the present disclosure. In some embodiments, the shape of the walls is dictated by the additional functionality desired for an adapter.

In some embodiments, an adapter incorporates electrical functionality, such as indicator lights, electrodes/elements for other types of sensing (e.g., capacitive, acoustic, optical, magnetic, tactile, *etc.), etc.* For example, in some embodiments adapter walls include capacitive electrodes that enable imaging of the content of the reservoirs via electrical impendence tomography, as described in the '121 application, or by capacitive sensing schemes described in the '779 application. Electrical connection to electrical devices disposed on the walls of an adapter can be established via, for example, electrical connectors, proximity contacts, and the like.

It should be noted that, in some embodiments, the height of the walls of an adapter is selected based on their desired functionality beyond their light-blocking capability. For example, although walls **512** and **604** have height **h1** (i.e., the same height as blisters **210),** the walls can be shorter or longer than the height of the blisters to control the vertical position of blister card **122,** secure the blister card in place, and/or optimize sensing capability. For example, in embodiments in which walls **512** and/or **604** include electrodes to effect capacitive sensing, a wall height that locates the bottom of the blisters in close proximity to their respective capacitive sensors can be beneficial.

In some embodiments, not all of the electronic circuitry of a smart case is located in electronics module **104.** Instead, in some embodiments, substrate **506** (or seat **312** or lid **304** or receiver **112** or any combination thereof) includes some electronics functionality beyond just that associated with their respective sensor systems. This is particularly advantageous for modular smart cases in accordance with the present disclosure.

Furthermore, in some embodiments, a photodiode-based sensor system includes a photodiode array that has more elements than the number of reservoirs on the blister card for which it is intended. As discussed below, a sensor system having higher sensor density than the reservoirs can, for example, provide more detection data per reservoir, enable the use of the same smart case with blister cards having different sizes and/or formats, and more. For smart cases intended to be operative for a set of blister cards having different configurations, it is preferable that the density of photodiodes of a photodiode-based sensor system be equal to or greater than the highest reservoir density of any of the set of blister cards.

**FIG. 6B** depicts detection module **600** with the addition of optional cover **606.**

Cover **606** is a thin decorative layer configured to hide sensor system **502.** Cover **606** includes apertures **608,** which are formed in the layer to enable light to pass through it and be received by photodiodes **408.** In the depicted example, apertures 608 are through holes; however, a wide range of apertures are within the scope of the present disclosure, such as transparent regions that function as windows, *etc.* In some embodiments, apertures **608** include one or more optical elements (e.g., lenses, diffraction gratings, blazed gratings, *etc.)* for facilitating the detection of light at photodiodes **408.**

It should be noted that the material and structure of cover **606** are based on the sensing technology used in sensor system **502.** For example, for aesthetics it is often desirable to hide sensor system **502;** therefore, in such instances, optically opaque material is desirable. When sensor system **502** employs capacitive sensing, however, the material of cover **606** must be electrically insulating and thin, and the cover would not typically include apertures **608.**

As will be appreciated by one of ordinary skill in the art, smart medicine cases (or other smart packages) known in the prior art are configured for operation with only one particular blister card configuration and will not function for blister cards having different configurations. For the purposes of this Specification, including the appended claims, the **"configuration"** of a blister card is defined as the size and shape of the blister card, as a whole, and its blister-card parameters, which include the number, size, shape, and inter-reservoir spacing between its reservoirs. It is an aspect of the present disclosure, however, that a smart case can be configured to accept any of a set of blister cards having different configurations by employing an appropriate adapter, where necessary, for each blister card of the set.

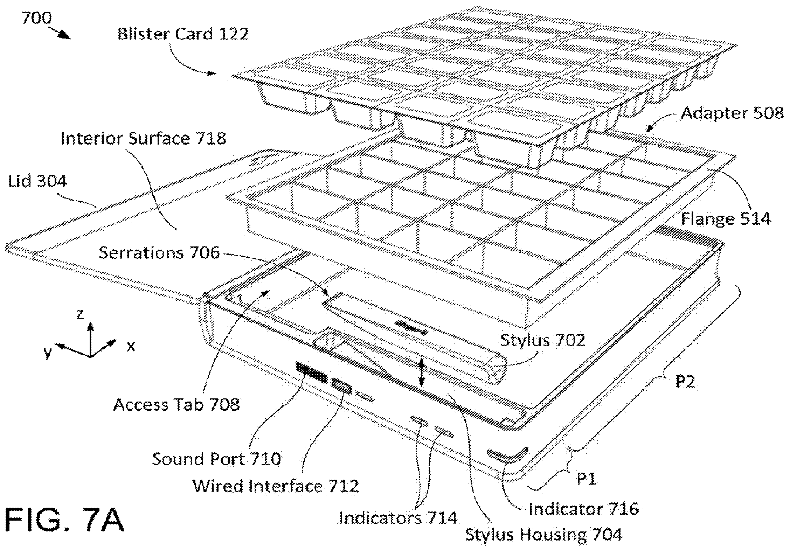

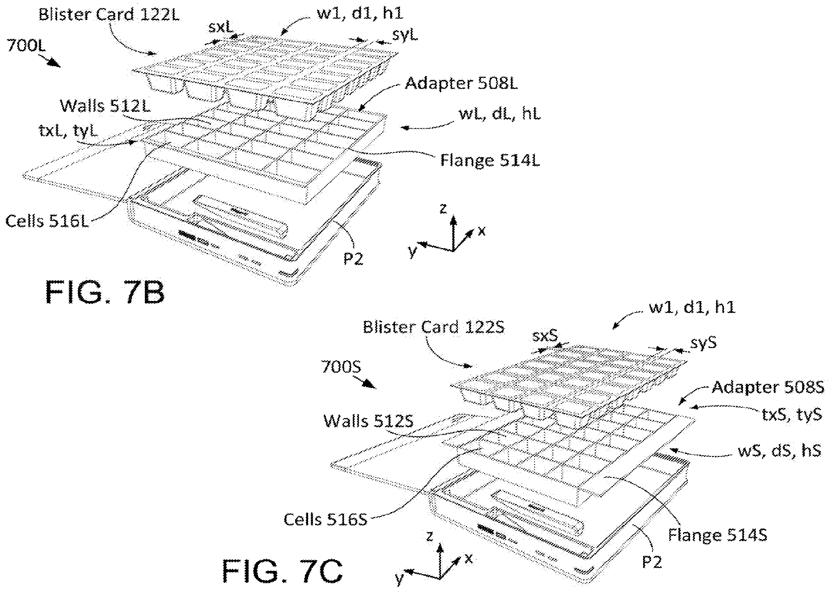

**FIG. 7A** depicts a schematic drawing of an exploded perspective view of the salient features of a reconfigurable smart case in accordance with the present disclosure. Case **700** is analogous to case **500;** however, case **700** includes optional stylus **702,** stylus housing **704,** access tab **708,** and more, all of which are located in body portion **P1.** In the example depicted in FIG. 7A, case **700** is configured to monitor that state of blister card **122,** as discussed above.

Stylus **702** is designed for piercing dispensing regions **212** and scooping out content **208** from reservoirs **206.** Although this function can often be done by the user using a finger or another object (e.g., fork, spoon, *etc.),* stylus **702** can provide significant advantages. For example, stylus **702** includes optional serrations **706** to facilitate puncture and removal of lidding film **204** in dispensing regions **212.**

In the depicted example, body portion **P1** also includes access tab **708** to facilitate removal of adapter **508.** In some embodiments, adapter **508** includes an opposing recess in flange **514** to further facilitate removal of blister card **122.**

FIG. 7A also depicts additional exemplary user interfaces, such as sound port **710,** wired interface **712,** and visual indicators **714.** Non-limiting examples of user interfaces suitable for use in smart cases in accordance with the present disclosure are described in detail in the '874, '121, '779, and '021 applications. It should be noted that the interfaces depicted, and their locations, are merely exemplary and many different interfaces, at any suitable locations, can be included in a smart case without departing from the scope of the present disclosure.

Sound port **710** includes perforations and/or holes that allow sound to travel into and out of case **700.** They are used in conjunction with microphones, speakers, sirens, buzzers, *etc.* for verbal interaction with the user, sound alerts and any other acoustic interfacing.

Wired interface port **712** is a conventional connection (e.g., USB, Micro USB, *etc.)* for enabling wired two-way communication and/or charging of an energy storage unit.

Indicators **714** are display elements (e.g., light-emitting diodes, incandescent lamps, liquid-crystal elements, etc.) for providing visual alerts and/or status indications to the user (e.g., visual dosing time reminder, the case is functioning correctly, energy storage unit is charging/charged, present adherence status, an issue must be addressed, *etc.).*

Indicator **716** is a visual display element that is configured to flash to gain the attention of the user. Indicator **716** is optionally included to assist a user who is hearing impaired in recognizing that interaction with case **700** is needed (e.g., a dosing window is happening or imminent, a dose has been missed, a dose has been improperly dispensed, *etc.).*

In some embodiments, one or more user interfaces are disposed on interior surface **718** of lid **304.** Such user interfaces can include, for example, one or more displays, touch switches, mirrors, tabs/holders (e.g., for holding papers, labels, tablets, a smart phone, and the like), *etc.* to implement desired functionalities and features.

**FIGS. 7B-C** depict schematic drawings of exploded perspective views of smart case **700** as configured for use with blister cards that are larger and smaller, respectively, than blister card **122.**

Case **700L** represents smart case **700** as configured to accept and monitor blister card **122L.** Case **700L** includes case **700** and adapter **508L.**

Blister card **122L** has the same outer dimensions (i.e., overall size and shape) as blister card **122;** however, at least one of the blister-card parameters **(wL, dL, hL)** of blister card **122L** is different than those of blister card **122.** Specifically, in the depicted example, blister card **122L** includes the same reservoirs (i.e., reservoirs **206)** as blister card **122;** therefore, **wL=w1, dL=d1,** and **hL=h1).** However, the inter-reservoir spacings, **sxL** and **syL,** of the reservoirs is greater along each of the x- and y-dimensions than the inter-reservoir spacings of blister card **122.** As a result, blister card **122L** is characterized by a blister region **218L** (not shown), which has a larger perimeter than blister region **218.**

Adapter **508L** includes flange **514L,** which has the same outer perimeter (OP1) as adapter **508,** thereby enabling adapter **508L** to seat securely in body portion **P2.** Since the inner dimensions of flange **514L** are configured to accept blister card **122L,** flange **514L** has an inner perimeter **IPL** (not shown) that is larger than that of flange **514.** As a result, flange **514L** is narrower than flange **514** - in fact, it is non-existent along two of the outer edges of adapter **508L.**

The dimensions and spacing of walls **512** of adapter **508L** are selected based on the parameters of blister card **122L.** Since reservoirs **206** are characterized by a larger inter-reservoir spacing in blister card **122L,** walls **512L** are thicker.

In similar fashion, case **700S** includes case **700** and adapter **508S,** where adapter **508S** is configured to receive blister card **122S.** Blister card **122S** also has the same outer dimensions (i.e., overall size and shape) as blister card **122;** however, at least one of the blister-card parameters **(wS, dS, hS)** of blister card **122S** is different than those of each of blister cards **122** and **122L.** Specifically, in the depicted example, blister card **122S** includes the same reservoirs (i.e., reservoirs **206)** as blister card **122;** therefore, **wS=w1, dS=d1,** and **hS=h1).** however, the inter-reservoir spacings, **sxS** and **syS,** of the reservoirs is smaller along each of the x- and y-dimensions than the inter-reservoir spacings of blister card **122.** As a result, blister card **122S** is characterized by a blister region **218S** (not shown), which has a smaller perimeter than blister region **218.**

As a result, adapter **508S** includes flange **514S,** which has the same outer dimensions as adapter **508,** thus enabling it to seat securely in body portion **P2.** The inner perimeter **IPS** (not shown) of flange **514S** is configured to accept blister card **122S,** thus it is smaller than that of flange **514** and flange **514S** is wider than flange **514.**

It should be noted that an adapter such as adapter **508S** can be used to accommodate any blister card whose outer dimensions are no larger than those of blister card **122.**

Since reservoirs **206** are characterized by a smaller inter-reservoir spacing in blister card **122S,** walls **512S** are thinner than walls **512.**

Since adapters can be made rather inexpensively (e.g., with plastic injection molding), they provide an inexpensive way to accommodate a myriad of blister cards in the same smart case.

It should be noted that the examples provided above merely represent a few of a wide range of approaches for enabling a smart case to accommodate a wide range of blister card sizes and/or reservoir geometries within the scope of the present disclosure.

To accommodate blister cards having different configurations of reservoirs, a case must include a sensor system that is capable of monitoring the contents of each individual reservoir regardless of its position within the blister card. Sensor systems having a one-to-one correspondence to the distribution of reservoirs in a given blister card, for example, are typically not suitable for use with any blister card other than the type for which it is designed.

As noted briefly above, in some embodiments, a photodiode-based sensor system preferably includes a plurality of photodiodes that has a density greater than the highest reservoir density of any blister card for which the sensor system is operative. Specifically, the number and density of the photodiodes is preferably chosen such that there exists at least one photodiode within the optically isolated reservoir locations for each blister card of the set of blister cards with which the smart case is intended to operate. There is, however, a special condition that allows using the same number of photodiodes as reservoirs for different blister cards. For this special condition, the different blister cards must have: (1) the same number of reservoirs; and (2) a common area of overlap between their respective blister cards. For a set of such blister cards, a single arrangement of photodiodes having a one-to-one correspondence with the reservoirs could be provided such that each photodiode resides in the overlap area of a corresponding reservoir of each of the different blister cards.

In some embodiments, a smart case employs a sensor system that includes a focal plane array that can receive light and substantially image all of the reservoirs on a blister card, regardless of their location. Such a sensor system would be able to monitor virtually any reservoir arrangement.

It should be noted that a similar strategy can be used with sensor systems based on sensing technologies other than optical sensing, such as resistance sensing, impedance sensing, capacitive sensing, acoustic sensing, tactile sensing, *etc.* described in detail in the '874, '121, '779, and '021 applications. In fact, a highly dense sensing system can enable identification of the geometry of an unknown blister card inserted into a smart case.

Furthermore, artificial intelligence and machine learning can be used to determine the geometry and content of a blister/blister card, thereby allowing identification of an unknown blister card and its features, as well as those of an adapter loaded into the smart case.

It should be noted that when a dispensing region is pierced to enable removal of the contents of a reservoir, a downward force is typically exerted on the blister card. In some embodiments, therefore, adapter **508** includes features that function as detents to hold blister card **122** in place. As noted above, one or more surfaces of walls **512** can also be provided with a "tacky" surface that mitigates slippage of the blister card relative to the adapter.

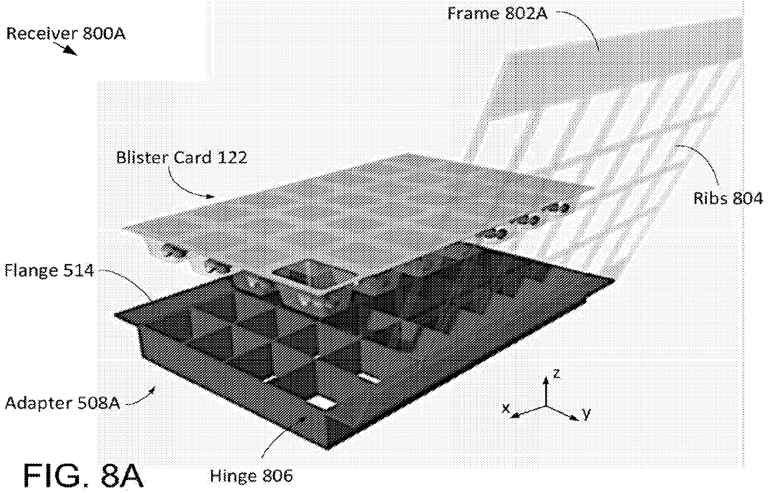

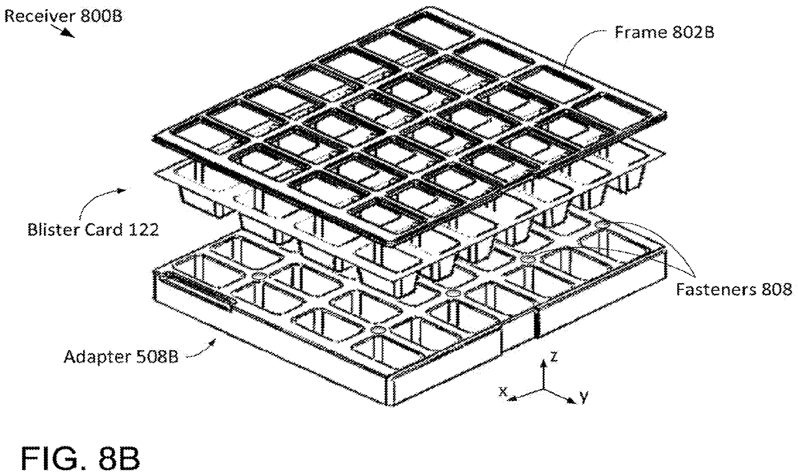

**FIGS. 8A-B** depict exploded schematic perspective drawings of two alternative receivers in accordance with the present disclosure. Receiver **800A** includes seat **510** (not shown), adapter **508A,** and frame **802A.**

Frame **802A** includes ribs **804,** which are configured to lie between reservoirs **206** of blister card **122** when the frame is latched in place.

Frame **802A** is attached to adapter **508A** by hinge **806,** which is located along the edge of the adapter where it meets frame **802A.** Frame **802A** also includes a conventional latch (not shown) configured to attach to flange **514** and secure frame **802A** over blister card **122** to hold the blister card in place.

Receiver **800B** is analogous to receiver **800A;** however, in receiver **800B,** frame **802B** is magnetically attached to adapter **508B** instead of via a hinge.

Each of frame **802B** and adapter **508B** includes a fastener **808** that is configured to hold the frame in place once it is placed over the blister card, which locks the blister card in place as well. In the depicted example, fastener **808** is a permanent magnet; however, other conventional fasteners (e.g., Velcro, mechanical latches, electromagnetic fasteners, *etc.)* can be used as fastener **808** without departing from the scope of the present invention.

It should be noted that some blister cards are designed to make them more child proof by using a multi-layer lidding film that is difficult to rupture or tear. Typically, the layers include a metal foil (e.g., aluminum) on the inside, paper on the outside, and a thin polymer layer sandwiched in between the metal and paper. In such blister card, peeling the lidding film off is a central step to accessing the content of a reservoir. Unfortunately, this peeling action applies an upward force on the blister card; however, when frames **802A** and **802B** are latched, the frame keeps the blister card in place.

Accordingly, embodiments in accordance with the present disclosure are suitable for use with such "peel-away" blister cards, even if a user elects to remove the blister card from the seat, tear off a dose *(i.e.,* one or more reservoirs) along the card's perforation lines and place the card back into the seat. It should be noted that if the user is expected to always remove the peel-away card as just described, the seat need not include a frame, simplifying card removal and re-insertion of the card.

As long as a blister card is properly located in the receiver of a smart case in accordance with the present disclosure, the blister card can be interrogated for reservoirs removed from the card, empty reservoirs, partially empty reservoirs and untouched reservoirs. It should be noted that, for a smart case comprising a photodiode-based sensing system, a reservoir region removed from a blister card results in more light being incident on its respective photodiode or photodiodes than an empty blister remaining on the card.

One skilled in the art will appreciate that a reservoir can be considered a special shape of a bottle, vessel, vial, tub, tube, pouch, can, cartridge, and the like. Accordingly, the application of smart cases in accordance with the present disclosure is not limited to only blister-card formats.

Furthermore, the type of material contained in a content compartment can dictate the preferred type of sensing technology employed in a smart case. For example, a content compartment might contain loose pills, liquids, gels, syrups, suspensions, powders, pastes, creams, strips, vapor, spray, and the like, that is at least partially optically transparent. As a result, a sensing technology other than optical detection might be dictated. In some instances, a capacitive sensing scheme (including electrical impedance tomography) would be preferred if, for example, the content were electrically insulating. If the content is electrically conductive, electrical resistance or impedance sensing (including electrical resistance or impedance tomography) might be preferred. In some instances, acoustic sensing might be preferred.

It is to be understood that the disclosure teaches just some exemplary embodiments and that many variations of the invention can easily be devised by those skilled in the art after reading this disclosure and that the scope of the present invention is to be determined by the following claims.

## Claims

1. System including a smart case (500) for monitoring the state of a blister card (122) of a set of blister cards having different configurations, the blister card comprising a plurality of blisters (210) and a lidding film (204) having a plurality of dispensing regions (212), the plurality of blisters (210) and the plurality of dispensing regions collectively defining a plurality of reservoirs (206) that contain content (208), wherein the plurality of blisters collectively define a blister region (218),
wherein the smart case comprises a detection module (106, 500) that includes:
a receiver (112, 404, 504, 800A, 800B) that is located in a first body portion (P2) of the smart case;
an adapter (508, 508L, 508S, 508A, 508B, 602) having a flange (514, 514S, 520, 520L) that has an outer perimeter (OP1) and an inner perimeter (IP1), the outer perimeter being based on the dimensions of the first body portion and the inner perimeter being based on the blister region of a blister card to be received by the adapter; and
a sensor system (114, 406, 502) that is configured to monitor the state of at least one of the lidding film (204) and the content of the blister card to be received by the adapter,
wherein the system is **characterized by** that it comprises a plurality of different adapters, each of which can be mounted in the same smart case (500) to enable it to accept any of the set of blister cards,
wherein, by the inclusion of the adapter in the receiver, the receiver is configured to locate and secure the blister card of the set of blister cards by employing the adapter being appropriate for the blister card, wherein the adapter is one of the plurality of different adapters usable with the smart case, each adapter of the plurality of different adapters being removable from the smart case.

2. The system of claim 1 wherein the adapter further includes a plurality of walls (512) that define a plurality of cells (516), and wherein at least one dimension (tx1, ty1) of at least one wall of the plurality thereof (512) is based on an inter-reservoir spacing (sx1, sy1) of the blister card (122)to be received by the adapter, and further wherein at least one dimension (w2, d2, h2) of a cell of the plurality thereof is based on at least one dimension (w1, d1, h1) of a blister of the plurality of blisters of the blister card.

3. The system of claim 1 wherein the receiver is further configured to locate and secure a second blister card (122L) of the set of blister cards comprising a second lidding film and a second plurality of blisters that collectively define a second plurality of reservoirs that contain second content, and wherein the second plurality of blisters collectively define a second blister region (218L), and
wherein the adapter is a second adapter (508L) of the plurality of different adapters, the second adapter having a second flange (520L) that has the outer perimeter and a second inner perimeter (IPL) that is based on the second blister region;
wherein the sensor system is further configured to monitor the state of at least one of the second lidding film and the second content of the second blister card.

4. The system of claim 1 wherein the receiver of the smart case further includes a frame (802A) that is configured to removably attach to the adapter, wherein the frame (802A) and the adapter are dimensioned and arranged to immobilize the blister card (122) when the frame (802A) is attached to the adapter (508A).

5. The system of claim 4 wherein the frame (802A) and the adapter are attached via a hinge (806) or wherein at least one of the frame and the adapter includes a fastener (808) that is configured to hold the frame and the adapter in a first arrangement.

6. The system of claim 1, wherein the smart case (500) further comprises:
a plurality of connectors (316) that are arranged around the perimeter of the blister card (122) and configured to electrically couple with a lidding film when the blister card is located in the receiver (112, 800A), the sensor system comprising the plurality of connectors; and
an electronics module (104) that is configured to receive at least one signal (124) from the plurality of connectors and generate a tomographic image of the lidding film based on the plurality of electrical signals.

7. The system of claim 1 wherein the sensor system of the smart case (500) includes a plurality of sensors (408) that includes a first sensor (408-5C) configured to monitor the state of at least one of a first dispensing region (212-5C) of the plurality of dispensing regions and a first content (208-5C) of the content.

8. The system of claim 7 wherein the first sensor (408-5C) is selected from the group consisting of a capacitive sensor, a strain sensor, an optical sensor, an acoustic sensor, a tactile sensor, a resistance sensor, an impedance sensor, a thermal sensor, and a magnetic sensor.

9. The system of claim 7 wherein at least one sensor of the plurality thereof is disposed on a wall (512) of the adapter (508).

10. The system of claim 7 wherein the first sensor (408-5C) is a photodiode configured to receive light transmitted through a first reservoir (206-5C) when its respective first dispensing region (212-5C) is at least partially removed.

11. The system of claim 10 wherein the first reservoir (206-5C) is adjacent to a second reservoir (206-4C), and wherein the adapter (508) includes a wall (512) that is disposed between the first and second reservoirs, the wall being configured to block propagation of light from the second reservoir to the photodiode.

12. The system of claim 7 wherein the smart case is configured to operatively couple with each of a set of blister cards, and wherein the plurality of sensors (408) has a sensor density that is greater than or equal to the highest reservoir density of the set of blister cards.

13. The system of claim 1 wherein the sensor system includes a focal-plane array that is configured to image at least a portion of the blister card.

14. The system of claim 1 wherein the smart case (500) includes a housing (102) comprising a body (302), a lid (304), and a latch (308) for reversibly securing the lid and body in a closed configuration, wherein the latch is electrically actuatable.

## Patentansprüche

1. System mit einem intelligenten Etui (500) zur Überwachung des Zustands einer Blisterkarte (122) aus einem Satz von Blisterkarten mit unterschiedlichen Konfigurationen, wobei die Blisterkarte eine Vielzahl von Blistern (210) und eine Abdeckfolie (204) mit einer Vielzahl von Entnahmebereichen (212) umfasst, wobei die Vielzahl von Blistern (210) und die Vielzahl von Entnahmebereichen gemeinsam mehrere Reservoirs (206) definieren, die Inhalt (208) enthalten, wobei die Vielzahl von Blistern gemeinsam einen Blisterbereich (218) definieren,
wobei das intelligente Etui ein Erfassungsmodul (106, 500) umfasst, das folgendes beinhaltet:
einen Empfänger (112, 404, 504, 800A, 800B), der sich in einem ersten Körperabschnitt (P2) des intelligenten Etuis angeordnet ist;
einen Adapter (508, 508L, 508S, 508A, 508B, 602) mit einem Flansch (514, 514S, 520, 520L), der einen Außenumfang (OP1) und einen Innenumfang (IP1) besitzt, wobei der Außenumfang auf den Abmessungen des ersten Körperabschnitts basiert und der Innenumfang auf dem Blisterbereich einer vom Adapter aufnehmenden Blisterkarte; und
ein Sensorsystem (114, 406, 502), das so konfiguriert ist, dass es den Zustand zumindest entweder der Abdeckfolie (204) oder des Inhalts der Blisterkarte, die vom Adapter aufgenommen werden soll, überwacht,
wobei das System **dadurch gekennzeichnet ist, dass** es eine Vielzahl verschiedener Adapter umfasst, von denen jeder in dasselbe intelligente Etui (500) eingesetzt werden kann, um die Aufnahme einer beliebigen Blisterkarte aus dem Satz von Blisterkarten zu ermöglichen,
wobei durch das Einsetzen des Adapters in den Empfänger dieser so konfiguriert ist, dass er die Blisterkarte aus dem Satz von Blisterkarten lokalisiert und sichert, indem er den für die Blisterkarte geeigneten Adapter verwendet, wobei der Adapter einer der Vielzahl von verschiedenen Adaptern ist, die mit dem intelligenten Etui verwendet werden können, und jeder Adapter aus der Vielzahl der verschiedenen Adapter aus dem intelligenten Etui wieder entnehmbar ist.

2. System nach Anspruch 1, wobei der Adapter ferner eine Vielzahl von Wänden (512) umfasst, die eine Vielzahl von Zellen (516) definieren, und wobei mindestens eine Abmessung (tx1, ty1) mindestens einer Wand aus der Vielzahl davon (512) auf einem Abstand (sx1, sy1) zwischen den Reservoirs der Blisterkarte (122) basiert, die von dem Adapter aufgenommen werden soll, und wobei ferner mindestens eine Abmessung (w2, d2, h2) einer Zelle der Vielzahl von Zellen auf mindestens einer Abmessung (w1, d1, h1) eines Blisters der Vielzahl von Blistern der Blisterkarte basiert.

3. System nach Anspruch 1, wobei der Empfänger ferner so konfiguriert ist, dass er eine zweite Blisterkarte (122L) aus dem Satz von Blisterkarten, die eine zweite Abdeckfolie und eine zweite Vielzahl von Blistern umfasst, die gemeinsam eine zweite Vielzahl von Reservoirs bilden, die zweiten Inhalt enthalten, lokalisiert und sichert, und wobei die zweite Vielzahl von Blistern gemeinsam einen zweiten Blisterbereich (218L) bilden, und
wobei der Adapter ein zweiter Adapter (508L) aus der Vielzahl verschiedener Adapter ist, wobei der zweite Adapter einen zweiten Flansch (520L) aufweist, der den Außenumfang und einen zweiten Innenumfang (IPL) aufweist, der auf dem zweiten Blisterbereich basiert;
wobei das Sensorsystem ferner so konfiguriert ist, dass es den Zustand mindestens einer der zweiten Abdeckfolie und des zweiten Inhalts der zweiten Blisterkarte überwacht.

4. System nach Anspruch 1, wobei der Empfänger des intelligenten Etuis ferner einen Rahmen (802A) umfasst, der so ausgelegt ist, dass er lösbar an dem Adapter befestigt werden kann, wobei der Rahmen (802A) und der Adapter so dimensioniert und angeordnet sind, dass sie die Blisterkarte (122) fixieren, wenn der Rahmen (802A) an dem Adapter (508A) befestigt ist.

5. System nach Anspruch 4, wobei der Rahmen (802A) und der Adapter über ein Scharnier (806) verbunden sind oder wobei zumindest entweder der Rahmen oder der Adapter ein Befestigungselement (808) umfasst, das so ausgelegt ist, dass es den Rahmen und den Adapter in einer ersten Anordnung hält.

6. System nach Anspruch 1, wobei das intelligente Etui (500) ferner umfasst: eine Vielzahl von Anschlüssen (316), die um den Umfang der der Blisterkarte (122) angeordnet und so ausgelegt sind, dass sie eine elektrische Verbindung mit einer Abdeckfolie herstellen, wenn sich die Blisterkarte in der Aufnahme (112, 800A) befindet, wobei das Sensorsystem die Vielzahl von Anschlüssen umfasst; und
ein Elektronikmodul (104), das so ausgelegt ist, mindestens ein Signal (124) von der Vielzahl von Anschlüssen zu empfangen und auf der Grundlage der Vielzahl von elektrischen Signalen ein tomographisches Bild der Abdeckfolie zu erzeugen.

7. System nach Anspruch 1, wobei das Sensorsystem des intelligenten Etuis (500) eine Vielzahl von Sensoren (408) umfasst, zu denen ein erster Sensor (408-5C) gehört, der so konfiguriert ist, dass er den Zustand mindestens eines der folgenden Elemente überwacht: eines ersten Ausgabebereichs (212-5C) der Vielzahl von Ausgabebereichen oder eines ersten Inhalts (208-5C) des Inhalts.

8. System nach Anspruch 7, wobei der erste Sensor (408-5C) aus der Gruppe ausgewählt ist, die aus einem kapazitiven Sensor, einem Dehnungssensor, einem optischen Sensor, einem akustischen Sensor, einem taktilen Sensor, einem Widerstandssensor, einem Impedanzsensor, einem thermischen Sensor und einen magnetischen Sensor besteht.

9. System nach Anspruch 7, wobei mindestens ein Sensor aus der Vielzahl davon an einer Wand (512) des Adapters (508) angeordnet ist.

10. System nach Anspruch 7, wobei der erste Sensor (408-5C) eine Fotodiode ist, die so konfiguriert ist, dass sie Licht empfängt, das durch ein erstes Reservoir (206-5C) hindurchtritt, wenn dessen jeweiliger erster Abgabebereich (212-5C) zumindest teilweise entfernt ist.

11. System nach Anspruch 10, wobei das erste Reservoir (206-5C) an ein zweites Reservoir (206-4C) angrenzt und wobei der Adapter (508) eine Wand (512) aufweist, die zwischen dem ersten und dem zweiten Reservoir angeordnet ist, wobei die Wand so ausgelegt ist, dass sie die Ausbreitung von Licht vom zweiten Reservoir zur Fotodiode blockiert.

12. System nach Anspruch 7, wobei das intelligente Gehäuse so konfiguriert ist, dass es mit jeder eines Satzes von Blisterkarten funktionsfähig gekoppelt werden kann, und wobei die Vielzahl von Sensoren (408) eine Sensordichte aufweist, die größer oder gleich der höchsten Reservoirdichte der Gruppe von Blisterkarten ist.

13. Das System nach Anspruch 1, wobei das Sensorsystem ein Fokalebenen-Array umfasst, das so konfiguriert ist, dass es zumindest einen Teil der Blisterkarte abbildet.

14. System nach Anspruch 1, wobei das intelligente Gehäuse (500) ein Gehäuse (102) umfasst, das aus einem Körper (302), einem Deckel (304) und einer Verriegelung (308) zum reversiblen Sichern des Deckels und des Körpers in einer geschlossenen Konfiguration besteht, wobei die Verriegelung elektrisch betätigbar ist.

## Revendications

1. Système comportant un boîtier intelligent (500) permettant de surveiller l'état d'une plaquette alvéolée (122) d'un ensemble de plaquettes alvéolées ayant différentes configurations, la plaquette alvéolée comprenant une pluralité d'alvéoles (210) et un film de recouvrement (204) ayant une pluralité de zones de distribution (212), la pluralité d'alvéoles (210) et la pluralité de zones de distribution définissant collectivement une pluralité de réservoirs (206) qui contiennent un contenu (208), dans lequel la pluralité d'alvéoles définissent collectivement une zone d'alvéoles (218),
dans lequel le boîtier intelligent comprend un module de détection (106, 500) qui comporte :
un récepteur (112, 404, 504, 800A, 800B) qui est situé dans une première partie de corps (P2) du boîtier intelligent ;
un adaptateur (508, 508L, 508S, 508A, 508B, 602) ayant une bride (514, 514S, 520, 520L) qui a un périmètre externe (OP1) et un périmètre interne (IP1), le périmètre externe étant basé sur les dimensions de la première partie de corps et le périmètre interne étant basé sur la zone d'alvéoles d'une plaquette alvéolée devant être reçue par l'adaptateur ; et
un système de capteurs (114, 406, 502) qui est configuré pour surveiller l'état d'au moins un élément parmi le film de recouvrement (204) et le contenu de la plaquette alvéolée devant être reçue par l'adaptateur,
dans lequel le système est **caractérisé en ce qu'**il comprend une pluralité d'adaptateurs différents, chacun peut être monté dans le même boîtier intelligent (500) pour lui permettre d'accepter une quelconque plaquette parmi l'ensemble de plaquettes alvéolées,
dans lequel, par l'inclusion de l'adaptateur dans le récepteur, le récepteur est configuré pour localiser et sécuriser la plaquette alvéolée de l'ensemble de plaquettes alvéolées en faisant appel à l'adaptateur approprié pour la plaquette alvéolée, dans lequel l'adaptateur est un élément parmi la pluralité d'adaptateurs différents utilisables avec le boîtier intelligent, chaque adaptateur de la pluralité d'adaptateurs différents pouvant être retiré du boîtier intelligent.

2. Système selon la revendication 1, dans lequel l'adaptateur comporte en outre une pluralité de parois (512) qui définissent une pluralité de cellules (516), et dans lequel au moins une dimension (tx1, ty1) d'au moins une paroi de la pluralité de celles-ci (512) est basée sur un espacement inter-réservoir (sx1, sy1) de la plaquette alvéolée (122) devant être reçue par l'adaptateur, et en outre dans lequel au moins une dimension (w2, d2, h2) d'une cellule de la pluralité de celles-ci est basée sur au moins une dimension (w1, d1, h1) d'une alvéole de la pluralité d'alvéoles de la plaquette alvéolée.

3. Système selon la revendication 1, dans lequel le récepteur est en outre configuré pour localiser et sécuriser une seconde plaquette alvéolée (122L) de l'ensemble de plaquettes alvéolées comprenant un second film de recouvrement et une seconde pluralité d'alvéoles qui définissent collectivement une seconde pluralité de réservoirs contenant un second contenu, et dans lequel la seconde pluralité d'alvéoles définissent collectivement une seconde zone d'alvéoles (218L), et
dans lequel l'adaptateur est un second adaptateur (508L) de la pluralité d'adaptateurs différents, le second adaptateur ayant une seconde bride (520L) qui a le périmètre externe et un second périmètre interne (IPL) qui est basé sur la seconde zone d'alvéoles ;
dans lequel le système de capteurs est en outre configuré pour surveiller l'état d'au moins un élément parmi le second film de recouvrement et le second contenu de la seconde plaquette alvéolée.

4. Système selon la revendication 1, dans lequel le récepteur du boîtier intelligent comporte en outre un cadre (802A) qui est conçu pour se fixer à l'adaptateur de manière à pouvoir être retiré, dans lequel le cadre (802A) et l'adaptateur sont dimensionnés et agencés pour immobiliser la plaquette alvéolée (122) lorsque le cadre (802A) est fixé à l'adaptateur (508A).

5. Système selon la revendication 4, dans lequel le cadre (802A) et l'adaptateur sont fixés par l'intermédiaire d'une charnière (806) ou dans lequel au moins un élément parmi le cadre et l'adaptateur comporte une fixation (808) qui est conçue pour maintenir le cadre et l'adaptateur dans un premier agencement.

6. Système selon la revendication 1, dans lequel le boîtier intelligent (500) comprend en outre :
une pluralité de connecteurs (316) qui sont agencés autour du périmètre de la plaquette alvéolée (122) et configurés pour se coupler électriquement avec un film de recouvrement lorsque la plaquette alvéolée est placée dans le récepteur (112, 800A), le système de capteurs comprenant la pluralité de connecteurs ; et
un module électronique (104) qui est configuré pour recevoir au moins un signal (124) de la pluralité de connecteurs et générer une image tomographique du film de recouvrement sur la base de la pluralité de signaux électriques.

7. Système selon la revendication 1, dans lequel le système de capteurs du boîtier intelligent (500) comporte une pluralité de capteurs (408) qui comporte un premier capteur (408-5C) configuré pour surveiller l'état d'au moins un élément parmi une première zone de distribution (212-5C) de la pluralité de zones de distribution et un premier contenu (208-5C) du contenu.

8. Système selon la revendication 7, dans lequel le premier capteur (408-5C) est choisi dans le groupe constitué d'un capteur capacitif, d'un capteur de contrainte, d'un capteur optique, d'un capteur acoustique, d'un capteur tactile, d'un capteur de résistance, d'un capteur d'impédance, d'un capteur thermique et d'un capteur magnétique.

9. Système selon la revendication 7, dans lequel au moins un capteur de la pluralité de ceux-ci est disposé sur une paroi (512) de l'adaptateur (508).

10. Système selon la revendication 7, dans lequel le premier capteur (408-5C) est une photodiode configurée pour recevoir de la lumière transmise à travers un premier réservoir (206-5C) lorsque sa première région de distribution (212-5C) respective est au moins partiellement retirée.

11. Système selon la revendication 10, dans lequel le premier réservoir (206-5C) est adjacent à un second réservoir (206-4C), et dans lequel l'adaptateur (508) comporte une paroi (512) qui est disposée entre les premier et second réservoirs, la paroi étant conçue pour bloquer une propagation de la lumière du second réservoir vers la photodiode.

12. Système selon la revendication 7, dans lequel le boîtier intelligent est configuré pour se coupler de manière fonctionnelle avec chaque plaquette d'un ensemble de plaquettes alvéolées, et dans lequel la pluralité de capteurs (408) a une densité de capteurs qui est supérieure ou égale à la densité de réservoir la plus élevée de l'ensemble de plaquettes alvéolées.

13. Système selon la revendication 1, dans lequel le système de capteurs comporte un réseau de plan focal qui est configuré pour imager au moins une partie de la plaquette alvéolée.

14. Système selon la revendication 1, dans lequel le boîtier intelligent (500) comporte un logement (102) comprenant un corps (302), un couvercle (304) et un loquet (308) pour sécuriser de manière réversible le couvercle et le corps dans une configuration fermée, dans lequel le loquet est actionnable électriquement.
